# EUROPEAN PATENT SPECIFICATION

(11) **EP 4 007 575 B1**
(45) Date of publication and mention of the grant of the patent: **27.09.2023**
(21) Application number: 20792979.5
(22) Date of filing: 16.10.2020
(51) Int. Cl.: A61K 31/4439, A61K 45/06, A61P 1/04, A61P 9/00, A61P 13/00, A61P 25/22

(54) **4-[5-[(RAC)-1-[5-(3-CHLOROPHENYL)-3-ISOXAZOLYL]ETHOXY]-4-METHYL-4H-1,2,4-TRIAZOL-3-YL]PYRIDINE FOR USE IN PREVENTION AND/OR TREATMENT OF STRESS**
4-[5-[(RAC)-1-[5-(3-CHLORPHENYL)-3-ISOXAZOLYL]ETHOXY]-4-METHYL-4H-1,2,4-TRIAZOL-3-YL]PYRIDIN ZUR VERWENDUNG BEI DER VORBEUGUNG UND/ODER BEHANDLUNG VON STRESS
4-[5-[(RAC)-1-[5-(3-CHLOROPHÉNYL)-3-ISOXAZOLYL] ÉTHOXY]-4-MÉTHYL-4 H-1,2,4-TRIAZOL-3-YL] PYRIDINE DESTINÉE À ÊTRE UTILISÉE DANS LA PRÉVENTION ET/OU LE TRAITEMENT DU STRESS

(30) Priority: 05.11.2019 SE 1951265
(43) Date of publication of application: 08.06.2022
(73) Proprietor: THULIN, Claes, 112 20 Stockholm (SE)
(72) Inventor: THULIN, Claes, 112 20 Stockholm (SE)
(74) Representative: Swea IP Law AB
(86) International application number: PCT/EP2020/079178
(87) International publication number: WO 2021/089300

(56) References cited:
- WO-A1-2007/040982
- WO-A1-2020/228973
- KÅGEDAL MATTS ET AL: "A positron emission tomography study in healthy volunteers to estimate mGluR5 receptor occupancy of AZD2066 - Estimating occupancy in the absence of a reference region", NEUROIMAGE, vol. 82, 11 May 2013 (2013-05-11), pages 160-169, XP028705566, ISSN: 1053-8119, DOI: 10.1016/J.NEUROIMAGE.2013.05.006
- CARLA MARADEY-ROMERO ET AL: "New and Future Drug Development for Gastroesophageal Reflux Disease", JOURNAL OF NEUROGASTROENTEROLOGY AND MOTILITY JAN 2014, vol. 20, no. 1, 31 January 2014 (2014-01-31), pages 6-16, XP055759860, KW ISSN: 2093-0879, DOI: 10.5056/jnm.2014.20.1.6

## Description

### Field of the invention

The present invention relates to compound 4-[5-[(rac)-1-[5-(3-Chlorophenyl)-3-isoxazolyl]ethoxy]-4-methyl-4*H*-1,2,4-triazol-3-yl]pyridine (TT00) and/or its *(1R)* or *(1S)* enantiomers (TT001 and TT02), or a pharmaceutical composition comprising said TT00, TT001 and/or TT002, for use in prevention and/or treatment of stress in combination with one or more disorders selected from the group consisting of operative gastroesophageal reflux, anxiety, ulcer, renal and vascular disorders, wound healing and inappetence in a dog, cat, horse or pig.

### Background of the invention and prior art

Induction of anaesthesia in mammals comprehends many levels of challenges. The general stress experiences by mammals, such as cats, pigs, dogs and horses can cause a necessary use of higher concentrations of pharmaceuticals to induce and maintain the anaesthesia.

During the recovery period, the patient again experiences discomfort, pain and stress. These conditions prolong the recovery and inactivity phase. A longer recovery period for the patient elevates the risk for vascular and renal events and reduces wound healing. It may also affect appetite, such that inappetence may occur.

Commonly used today in a surgical setting are anxiolytics and pain killers to handle pre- and post-operative stress and pain. Nonsteroidal anti-inflammatory drugs (NSAIDs) are commonly used. Typical side effect for NSAIDs in dogs are, vomiting, loss of appetite, depression, and diarrhea. Other serious side effects are gastrointestinal ulcer, liver/kidney failure and even death has been reported.

CG Abdallah, et al., Chronic Stress (Thousand Oaks). 2017 Feb;1. doi:10.1177 / 2470547017704763. Epub 2017 Jun 8, describes the difference between disorders pain and stress and they state that the definition of stress is very broad; for example, showing violent pictures and acute aversive stimuli-like acute pain can be both stressful but involve different physiology. Furthermore, release of cortisol and activation of the hippocampus are often observed following stress, but rarely seen after acute pain. A substance that may be effective against pain may very well not be effective against stress.

Christian L.M. et al., Stress and Wound Healing, Neuroimmunomodulation. 2006 ; 13(5-6): 337-346, doi.org/10.1159/000104862, discloses that it has become clear that stress can significantly slow wound healing: stressors ranging in magnitude and duration impair healing in humans and animals. For example, in humans, the chronic stress of caregiving as well as the relatively brief stress of academic examinations impedes healing. Similarly, restraint stress slows healing in mice. Effects of stress on wound healing and surgical recovery may be compounded by effects of physical pain, a common symptom of both laboratory wounding and surgery.

G.B.Glavin, et al., The neurobiology of stress ulcers, Brain Research Reviews, 16 (1991) 301-343, 1991 Elsevier Science Publishers B.V., doi.org/10.1016/0165-0173(91)90012-W, discloses that acute gastric lesions occur in both animal and man following exposure to a variety of stressors, including some that are difficult to quantify. In 1936, Selye published two short papers in which he reported the occurrence of certain physiological changes in response to a wide array of different stressors. These responses included adrenal hypertrophy, thymicolymphatic involution and acute ulcers of the stomach. These were to become the classic symptoms of the stress syndrome. At that time, Selye reported that immobilization of laboratory rats would produce this syndrome. Thus, in 1936 the link was made between stress and stomach ulceration as shown in figure 1 of the article. But it would take another 20 years before additional significant publications would emerge in this area. Mice subjected to restraint stress also had significantly higher levels of serum corticosterone as compared to unstressed controls. The elevation in serum corticosterone was found important for the delay in healing and the increased incidence of infection because when the stressed animals were treated with a glucocorticoid receptor antagonist (RU40555), their healing rates were equivalent to nonstressed animals. These results demonstrated that the suppressive effect of glucocorticoids on inflammatory activity at the wound site was a key factor linking stress and healing.

Lloyd JKF, et al., Minimising Stress for Patients in the Veterinary Hospital, Veterinary Sciences, 13 Apr 2017, 4(2) DOI: 10.3390/vetsci4020022, discloses the factors that worsen fear and elicit aggression in dogs also apply to cats. Dogs and cats can experience extreme stress through being separated from their owners and kept in a cage. These animals undergo physiological changes, such as increased heart rate and release of cortisol-both of which may be associated with negative feelings, such as fear and anxiety. In addition, stressed animals may not eat or drink adequately, which can delay recovery.

Balcome at al., Contemporary Topics 2004 by the American Association for Laboratory Animal Science. Vol 43. No.6 / November 2004 discloses that laboratory routines are associated with stress, and that animals do not readily habituate to them. The data suggest that significant fear, stress, and possibly distress are predictable consequences of routine laboratory procedures, and that these phenomena have substantial scientific and humane implications for the use of animals in laboratory research.

Immobilization alone or in combination with other factors, most commonly cold room temperature, has been extensively used to study pharmacological agents as modulators of gastric ulcer in search of therapeutic strategies. The most common procedures in current use are supine restraint in a cold (4°C) room or tube restraint plus partial immersion in room temperature (20°C) water. These techniques are chosen because they produce relatively large amounts of ulceration (averages of 15-40 mm of linear erosions are common) that are modifiable by a number of treatments both prior to and following the stress ulcer induction technique, manipulations such as prior stress challenges or rest conditions following the ulcerogenic stressor.

Nagy et al , Forced swimming test in mice: A review of antidepressant activity, February 2005, Psychopharmacology 177(3):245-55, who report that 'forced swim' in room-temperature water (23°C) for 5 hours also produces hemorrhagic gastric glandular lesions.

Saeid Golbidi, et al., Am J Physiol Heart Circ Physiol 308: H1476-H1498, 2015. Review First published April 17, 2015, discloses how chronic stress impacts the cardiovascular system. Atherosclerosis-promoting effects of forced swimming have been shown in a stress protocol consisting of restrain and forced swimming in rats fed a normal diet. This chronic stress model enhanced different aspects of atherosclerosis including increases in cholesterol and triglycerides blood levels, decreased HDL, increased oxidative stress, reduction of vascular elastic fibers, and boosted foam cell formation. Hypercholesterolemic and oxidative effects of stress remained 20 weeks after cessation of the stress protocol. The deleterious effects of forced swimming on cardiac function have also been shown in this experimental setting.

Marchon R.G, et al., Kidney Blood Press Res 2018;43:1919-1926, 2018 The Author(s). Published by S. Karger AG, Basel, doi.org/10.1159/000496004, disclose immediate and late effects of stress on kidneys of prepubertal and adult rats. The results show that prepubertal stressed animals showed reduced kidney weight and volume and increased cortical-medullar ratio in comparison to its control group when evaluated immediately. Furthermore, stressed prepubertal and adult animals evaluated immediately had reduced glomerular volumetric density. Most importantly, all stressed groups exhibited reduced number of glomeruli per kidney. Other analyzed parameters did not differ significantly. It is concluded that chronic stress induced before and after puberty led to irreversible glomerular loss, however, renal impairment was interrupted by removal of the stress stimuli.

In recent years, complication caused by reflux in a surgical setting for companion animals has been highlighted in "Prevalence of and risk factors for intraoperative gastroesophageal reflux and postanaesthetic vomiting and diarrhoea in dogs undergoing general anaesthesia, Journal of Veterinary Emergency and Critical Care 27(4) 2017, pp 397-408 doi: 10.1111/vec.12613". The authors conclude that there is high prevalence of reflux in dogs during anaesthesia. This is also the case in cats "Prevalence of Gastroesophageal Reflux in Cats During Anaesthesia and Effect of Omeprazole on Gastric pH, J Vet Intern Med 2017;31:734-742".

The realization that reflux is a common ailment in operation/surgery in mammals has opened a new field of investigational medicines. Rodriguez-Alarcon, C. A. et al. "Gastroesophageal reflux in anesthetized dogs: a review" In: Rev Colomb Cienc Pecu, 2015, Vol. 28, pp. 144-155, discloses the use of active ingredients such as ranitidine for reducing the post-operative instances of gastroesophageal reflux (GER). Ranitidine reduces acid production, which only leads to a lower pH in the stomach, but Ranitidine has no effect on the reflux itself.

Reflux may cause peptic ulcers and other complications and an effect on the recovery period of the mammals or patients after operation.

Although animal models in rodents may be predictive of the effect in man, no studies have been presented where rodent models predict the outcome in dogs. There are at the same time few models for larger animals, where these effects can be measured.

### Species metabolite comparison

Before a molecule becomes a drug candidate many things must be cleared. Two important steps that the molecule must pass is the species compatibility by metabolite profile evaluation.

In the non-clinical part of drug development, studies are designed to determine the metabolite profiles and characterize selected metabolites that are generated following incubations of a molecule in rat and human hepatocytes. The results obtained will be used to compare hepatic metabolism in man to that of the rodent model used in safety studies.

One method used is taking rat and human hepatocytes and incubated them with the molecule. The metabolite profiles in the supernatants will be recorded by liquid chromatography. Selected metabolites will be characterized by accurate mass spectrometry. There could be more than 10 different metabolites from the hepatic metabolism.

The metabolite profile should be similar between the two species. If this is not the case, then the predictability of rodent models for man is not obvious and most probably the molecule will not become a drug candidate.

It is also well known that for certain indications like inflammatory diseases the predictability of rodent models for man is very poor (PNAS | February 26, 2013 | vol. 110 | no. 9 | 3507-3512, Genomic responses in mouse models poorly mimic human inflammatory diseases).

This is also true for clinical pain. Unfortunately, there are many ways that the biology of rodents may fail to accurately predict the biology and pharmacology of clinical pain conditions in humans. (Blackburn -Munro 2004; Le Bars et al. 2001,Translational Pain Research: From Mouse to Man. Kruger L, Light AR, editors. Boca Raton, FL: CRC Press/Taylor & Francis; 2010. Chapter 17 Large Animal Models for Pain Therapeutic Development, Darrell A. Henze and Mark O. Urban).

Based on the difficulty to predict both the metabolism of a drug and the metabolite profile between species and the response in man based on rodent models, a person skilled in the arts will not be able to predict the efficacy and toxicity of a drug for one species based on data from another species unless specific studies are conducted to verify the activity of the compound.

### Examples of drugs that have different effect in different species

Tramadol is widely used as a pain killer with good efficacy in man, the effect in dogs however is not present. (J Am Vet Med Assoc. 2018 Feb 15;252(4):427-432. doi: 10.2460/javma.252.4.427., Lack of effectiveness of tramadol hydrochloride for the treatment of pain and joint dysfunction in dogs with chronic osteoarthritis., Budsberg SC, Torres BT, Kleine SA, Sandberg GS, Berjeski AK).

It is also known that tramadol has an adverse effect that can cause the dog to lose concept and balance, giving the impression that it is "high". There are also drugs that are used in man that are toxic to animals. An example of this is phenolic drugs, including acetaminophen and aspirin that should not be given to cats. (PLoS One. 2011 Mar 28;6(3):e18046. doi: 10.1371 / journal.pone. 0018046, Evolution of a major drug metabolizing enzyme defect in the domestic cat and other felidae: phylogenetic timing and the role of hypercarnivory. Shrestha B1, Reed JM, Starks PT, Kaufman GE, Goldstone JV, Roelke ME, O'Brien SJ, Koepfli KP, Frank LG, Court MH., and Author information Comparative and Molecular Pharmacogenomics Laboratory, Department of Molecular Physiology and Pharmacology, Tufts University School of Medicine, Boston, Massachusetts, United States of America. Life Sci. 2006 Nov 25;79(26):2463-73. Epub 2006 Oct 5).

The relatively large number of pharmaceuticals used during an operation makes it more difficult to optimize the recovery/recuperation period.

### Combination Drug Therapy (CDT)

There are several studies looking at CDT for pain management.

Mao et al. J. Pain, 2011, vol 12, pp 157-166, discloses various combination drug therapies for treatment of pain. Most therapies use an opiate combined with another pain killer. mGIuR's are not mentioned at all. Mao J. et al, review available data for CDT in man but there is no mentioning of animals, whatsoever. In Table 1, different combinations are listed and rated in terms of positive or negative results. In Table 1 from Mao et al, it is also mentioned that a combination of Tramadol and acetaminophen [oral] showed a positive and better pain relief with add-on drug in man. If this CDT was given to a cat, the toxicity might have killed it.

See also, Vet Rec. 2019 Oct 5;185(13):406. doi: 10.1136/vr.105009. Epub 2019 Jul 18., Randomised trial of perioperative tramadol for canine sterilisation pain management, and Meunier NV1, Panti A2, Mazeri S1, Fernandes KA3, Handel IG2, Bronsvoort BMC1, Gamble L3, Mellanby RJ2., Author information The Roslin Institute and Royal (Dick) School of Veterinary Studies, University of Edinburgh, Easter Bush, UK. Royal (Dick) School of Veterinary Studies, University of Edinburgh, Easter Bush, UK. Worldwide Veterinary Service, Cranborne, UK. Life Sci. 2006 Nov 25;79(26):2463-73. Epub 2006 Oct 5., cDNA cloning and characterization of feline CYP1A1 and CYP1A2., Tanaka N1, Miyasho T, Shinkyo R, Sakaki T, Yokota H., Author information Laboratory of Veterinary Biochemistry, Graduate School of Veterinary Medicine, Rakuno Gakuen University, Ebetsu, Hokkaido 069-8501, Japan.

There might be a general misconception that by just combining different drugs, there will be an additive effect. In Table 1 from Mao et al, one in four combinations do not give a positive result. There are even situations, where combinations are lowering the total efficacy of a treatment.

For a person skilled in the art, it should be obvious that clinical results in different species do not translate automatically to other species. Therefore, a person skilled in the arts should and will suggest an independent evaluation of efficacy and toxicity for each species. This is obviously also the position of The Medical Products Agency in most countries.

There is thus a challenge to shorten time for recuperation after operation, especially in mammal, such as dogs, pigs, cats and horses.
TT00, or 4-[5-[(rac)-1-[5-(3-Chlorophenyl)-3-isoxazolyl]ethoxy]-4-methyl-4*H*-1,2,4-triazol-3-yl]pyridine 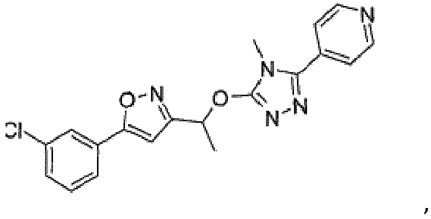 and
TT001, or 4-[5-[(1*R*)-1-[5-(3-Chlorophenyl)-3-isoxazolyl]ethoxy]-4-methyl-4*H*-1,2,4-triazol-3-yl]pyridine, AZD2066, CAS Number 934282-55-0, 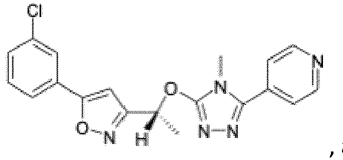 and
TT002, or 4-[5-[(1*S*)-1-[5-(3-Chlorophenyl)-3-isoxazolyl]ethoxy]-4-methyl-4*H*-1,2,4-triazol-3-yl]pyridine 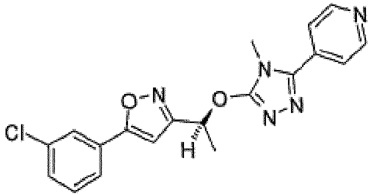 has a molecular formula C₁₉H₁₆ClN₅O₂ and a relative molecular mass of 381.8 (base).
TT00, especially TT001, is a selective non-competitive antagonist at the metabotropic glutamate receptors subtype 5 (mGluR5), being developed for oral treatment of chronic pain syndromes of neuropathic origin, anxiety and gastroesophageal reflux disease (GERD) in man.
TT00 has an effect on the mGluR5 receptor in the central nervous system, which may result in a central pain relief, reduced anxiety and reduced gastroesophageal reflux. The clinical development of TT001 was discontinued due to lack of effect of the substance in all indications.

In clinical development, dogs are used for toxicity studies, but no efficacy studies have been performed on these indications. Bibliographic searches do not find any articles on shortening recuperation, and treatment of anxiety and/or pain in dogs, pigs, cats or horses for said molecule.

WO2007/040982 discloses use of TT00, TT001 and/or TT002 in humans for treatment of neurological, psychiatric or pain disorders. IC50 bindings data in the mGluR receptor are shown as well as a study on the effect of TT00, TT001 or TT002 on TLESR in healthy dog in relation to food-intake. No results of the study are disclosed. No clinical data are present at all, especially no clinical data in association pain treatment after administration ofTT00, TT001 and/or TT002.

Rohof, et al., Aliment. Pharmacol. Ther. 2012, vol 35, pp 1231-1242, discloses a clinical study using TT001 to measure its effect on TLESR in healthy volunteers in association with food-intake. The results in figures 2 and 4 show that only a high dose of 13 mg (about 0.19 mg/kg) has some effect at 2 to 3 hours after food-intake, but that there was no significant effect on reflux prior to food intake, nor 1-2 hours after food intake. Some serious side effects associated with the high doses of 13 mg are mentioned. No clinical data are disclosed in association with pain treatment after administration of TT00, TT001 and/or TT002.

Furthermore, there are no published data relating to TT001 and the hepatic metabolism and metabolite profiles in rat and dog, and surely not for rat compared to cat and horse.

Richard H. P., The American Society for Pharmacology and Experimental Therapeutics JPET 315:711-721, 2005, disclose use of Fenobam, a metabotropic glutamate (mGlu)5 receptor antagonist, in anxiety in humans. The results show that at doses 10 (p <0.001) and 30 mg/kg (p <0.001) Fenobam significantly reversed stress-induced hyperthermia (SIH) in Mice. Studies performed with TT001 in humans showed no effect on anxiety in humans.

There is an unmet need for prevention and/or treatment of stress (surmenage) in combination with one or more disorders selected from operative gastroesophageal reflux, anxiety, ulcer, renal and vascular disorders, wound healing and/or inappetence in a mammal, such as dogs, pigs, cats or horses. There is a need for a treatment that has reduced side effect, such as gastrointestinal and renal toxicity.

### Summary of the invention

The present invention is defined by the claims. Any subject-matter falling outside the scope of the claims is provided for information only.

The present invention is directed to compounds 4-[5-[(rac)-1-[5-(3-Chlorophenyl)-3-isoxazolyl]ethoxy]-4-methyl-4*H*-1,2,4-triazol-3-yl]pyridine (TT00), 4-[5-[(1*R*)-1-[5-(3-Chlorophenyl)-3-isoxazolyl]ethoxy]-4-methyl-4*H*-1,2,4-triazol-3-yl]pyridine (TT001) and/or 4-[5-[(1*S*)-1-[5-(3-Chlorophenyl)-3-isoxazolyl]ethoxy]-4-methyl-4*H*-1,2,4-triazo1-3-yl] pyridine (TT002), or a pharmaceutically acceptable salt, solvate, isotope, or mixture thereof, for use in prevention and/or treatment of stress in combination with one or more disorders selected from the group consisting of operative gastroesophageal reflux, anxiety, ulcer, renal and vascular disorders, wound healing and inappetence in a dog, cat, horse or pig.

With reference to CG Abdallah, et al., Chronic Stress (Thousand Oaks). 2017 Feb;1, there is no reason to assume that a substance that works on pain could be used for a stress indication. Besides, the effect of TT001 for use in pain treatment has never been shown. TT001 has shown not to be effective for treatment of antidepression and anxiety. The new results however show an unexpected effect of the substance in the treatment of stress/surmenage.

In another aspect, the compound is a hydrochloride salt of TT001. In a further aspect, the compound is a sulphate salt of TT001. The mammal is a dog, cat, horse or pig.

In a normal operation/surgical setting, anesthetics, such as acepromazine, morphine and propofol and the like are used. Anesthetics, such as acepromazine have muscle relaxing properties that affect the lower esophageal sphincter (LES), causing more episodes of reflux during anesthesia. It may therefore be advantageous to administer the compounds TT00, TT001 and/or TT002 under operational conditions. Compounds TT00, TT001 and/or TT002, as defined above, are for use in prevention and/or treatment of surmenage/stress in a mammal under operational conditions. In another aspect, compounds TT00, TT001 and/or TT002, as defined above, are for use in prevention and/or treatment of surmenage/stress in a dog, cat, horse or pig treated with one or more muscle relaxing drugs. In a further aspect, compounds TT00, TT001 and/or TT002, as defined above, are for use in prevention and/or treatment of surmenage/stress in a dog, cat, horse or pig under operational conditions and treated with one or more muscle relaxing drugs. The compounds as defined above, are for use in prevention of surmenage/stress in a dog, cat, horse or pig. Said compounds may be administered before, during and/or after an operation on the mammal. In one aspect, said compounds are administered prior to an operation. It is advantageous when the mammal is relaxed/not stressed prior to the operation. Animals will become easier to handle before the operation when stress is reduced. Also, the effect on gastroesophageal reflux will be reduced because the compound TT001 may reduce this reflux. In another aspect, said compounds are administered after an operation. It is advantageous when the mammal is relaxed after the operation. A reduced level of stress in a mammal after operation is believed to reduce stress related complications that may occur due to stress after an operation, such as stomach disorders, brain damages, renal and/or vascular disorders, wound healing and/or inappetence.

In one aspect, compounds TT00, TT001 and/or TT002, as defined above are for use in prevention and/or treatment of surmenage/stress in combination with prevention and/or treatment of ulcer, renal and/or vascular disorders. In one aspect, compounds TT00, TT001 and/or TT002, as defined above are for use in prevention and/or treatment of surmenage/stress in combination with prevention and/or treatment of ulcers. In one aspect, compounds TT00, TT001 and/or TT002, as defined above are for use in prevention and/or treatment of surmenage/stress in combination with prevention and/or treatment of renal and/or vascular disorders. In another aspect, the mammal is under operational conditions and/or treated with one or more muscle relaxing drugs. The compounds as defined above, are for use in prevention of surmenage/stress in a mammal. As mentioned above, disorders like ulcer, renal and/or vascular disorders may occur in stressed mammals or in connection to an operation. An mGluR antagonist like compounds TT00, TT001 and/or TT002 is believed to be able to prevent the occurrence and/or to treat these disorders, especially in stressed mammals.

In one aspect, compounds TT00, TT001 and/or TT002, as defined above, are for use in prevention and/or treatment of surmenage/stress in combination with prevention and/or treatment of (poor) wound healing. In one aspect, compounds TT00, TT001 and/or TT002, as defined above, are for use in prevention and/or treatment of surmenage/stress in combination with prevention and/or treatment of inappetence. In another aspect, the mammal is under operational conditions and/or treated with one or more muscle relaxing drugs. The compounds as defined above, are for use in prevention of surmenage/stress in a mammal. As mentioned above, disorders like poor wound healing and/or inappetence may occur in stressed mammals or in connection to an operation. Two important parts of recovery after an operation/surgery are that the wound heals quickly and without complications and that the mammal starts eating and drinking. These two conditions, wound healing and feed intake, are controlled by the stress level of the mammal. An mGluR antagonist like compounds TT00, TT001 and/or TT002 is believed to be able to prevent the occurrence and/or to treat these disorders, especially in stressed mammals.

In one aspect, compounds TT00, TT001 and/or TT002, as defined above, are for use in prevention and/or treatment of surmenage/stress in combination with prevention and/or treatment of operative gastroesophageal reflux. In another aspect, the mammal is under operational conditions and/or treated with one or more muscle relaxing drugs. The reflux may be pre-operative gastroesophageal reflux, intra-operative gastroesophageal reflux and/or post-operative gastroesophageal reflux. The compounds as defined above, are for use in prevention of surmenage/stress in a mammal. Said compounds may have a positive effect on gastroesophageal reflux, which may be increased in stressed mammals, especially under operational conditions. Therefore, an mGluR antagonist like compounds TT00, TT001 and/or TT002 is believed to be able to prevent the occurrence and/or to treat operative gastroesophageal reflux in a mammal, especially in stressed mammals.

In one aspect, compounds TT00, TT001 and/or TT002, as defined above, are for use in prevention and/or treatment of surmenage/stress in combination with anxiety. In another aspect, the mammal is under operational conditions and/or treated with one or more muscle relaxing drugs. Although compound TT001 has shown not to be effective for treatment of anxiety, it has been found to be effective in prevention and/or treatment of surmenage/stress. The compounds as defined above, are for use in prevention of surmenage/stress in a mammal. Therefore, an mGluR antagonist like compounds TT00, TT001 and/or TT002 is believed to be able to prevent the occurrence and/or to treat anxiety in a mammal, especially in stressed mammals.

In one aspect, compounds TT00, TT001 and/or TT002, as defined above, are for use in prevention and/or treatment of surmenage/stress in combination with prevention and/or treatment of operative gastroesophageal reflux and anxiety. In another aspect, the mammal is under operational conditions and/or treated with one or more muscle relaxing drugs. The compounds as defined above, are for use in prevention of surmenage/stress in a mammal. An mGluR antagonist like compounds TT00, TT001 and/or TT002 is believed to be able to also prevent the occurrence and/or to treat gastroesophageal reflux and anxiety in a mammal.

The mammal is a dog, cat, horse or pig in any of the uses or combination of uses mentioned above. In an aspect, the mammal is a dog. In another aspect, the mammal is a cat or a horse.

In some aspects, the compound is TT001, or hydrochloride or sulphate salt thereof.

In some aspects, the prevention and/or treatment of one or more disorders selected from operative gastroesophageal reflux, anxiety, ulcer, renal and vascular disorders, wound healing and/or inappetence is in a stressed mammal, i.e. in a mammal having elevated levels of release of cortisol and activation of the hippocampus.

In some aspects, prevention and/or treatment of pain is disclaimed. In some aspects, prevention and/or treatment of depression is disclaimed.

Compounds TT00, TT001 and/or TT002 show few side effects at the intended dose. They do not lose the potency over longer administration periods. It is believed that the compounds as defined above, would have a reduced potential for side effects compared to conventional drugs used in mammals today, such as NSAIDS. The compounds of the invention are believed to have a reduced gastrointestinal and renal toxicity.

The invention further relates to a pharmaceutical composition, comprising compounds TT00, TT001 and/or TT002, or a pharmaceutically acceptable salt, solvate, isotope, or mixture thereof, in the association with a pharmaceutically acceptable adjuvant, diluent or carrier, for use in prevention and/or treatment of surmenage/stress, in combination with one or more disorders selected from the group comprising or consisting of operative gastroesophageal reflux, anxiety, ulcer, renal and vascular disorders, wound healing and inappetence. In another aspect, the mammal is under operational conditions and/or treated with one or more muscle relaxing drugs. The compounds as defined above, are for use in prevention of surmenage/stress in a mammal. The reflux may be pre-operative gastroesophageal reflux, intra-operative gastroesophageal reflux and/or post-operative gastroesophageal reflux.

The invention also relates to a process for the preparation of a pharmaceutical composition, as defined above, which comprises mixing compounds TT00, TT001 and/or TT002, as defined above, with a pharmaceutically acceptable adjuvant, diluent or carrier. In one aspect, the compound is TT001, or hydrochloride or sulphate salt thereof.

Compounds TT00, TT001 and/or TT002 as defined above may be administered at a dose of 0.1 to 5.0 mg/kg, or 0.1 to 2.0 mg/kg, or 0.1 to 1.0 mg/kg per day. One aspect relates to a dosage regime, wherein compounds TT00, TT001 and/or TT002 as defined above, are administered to an mammal in a dose of 0.1 to 5.0 mg/kg once daily, or twice daily. In one aspect, the dosage regime is administration of compounds TT00, TT001 and/or TT002 as defined above, at a dose of 0.1 to 1.0 mg/kg once daily. In some aspects, the compound is TT001, or hydrochloride or sulphate salt thereof. In some aspects, the compound is TT001, or hydrochloride salt thereof. The shortening of a recuperation period, and prevention and/or treatment of related pathology defined herein may be applied as a sole therapy or may involve, in addition to said compounds, conjoint treatment with conventional therapy of value in preventing and treating one or more disease conditions referred to herein. Such conventional therapy may include one or more of the following categories of agents: NSAIDs and opiates.

Mao et al. J. Pain, 2011, vol 12, pp 157-166, discloses various combination drug therapies for treatment of pain. Most therapies use an opiate combined with another pain killer. mGIuR's are not mentioned at all for use in a combination therapy.

Such conjoint treatment may be achieved by way of the simultaneous, sequential or separate dosing of the individual components of the treatment. Such combination products employ compounds TT00, TT001 and/or TT002, as defined above, for use in any of the disorders mentioned above.

In one aspect, the invention relates to a pharmaceutical composition comprising (i) compounds TT00, TT001 and/or TT002, or a pharmaceutically acceptable salt, solvate or isotope or mixture thereof, (ii) an additional therapeutic agent, or a pharmaceutically acceptable salt or solvate thereof, and (iii) a pharmaceutically acceptable excipient, carrier or diluent, for use in prevention and/or treatment of surmenage/stress, in combination with one or more disorders selected from the group comprising or consisting of operative gastroesophageal reflux, anxiety, ulcer, renal and vascular disorders, wound healing and inappetence. The compounds as defined above, are for use in prevention of surmenage/stress in a mammal.

In another aspect, the invention relates to a pharmaceutical composition comprising (i) compounds TT00, TT001 and/or TT002, or a pharmaceutically acceptable salt, solvate or isotope or mixture thereof, and a pharmaceutically acceptable excipient, carrier or diluent (ii) an additional therapeutic agent, or a pharmaceutically acceptable salt or solvate thereof, and a pharmaceutically acceptable excipient, carrier or diluent for use in prevention and/or treatment of surmenage/stress, in combination with one or more disorders selected from the group comprising or consisting of operative gastroesophageal reflux, anxiety, ulcer, renal and vascular disorders, wound healing and inappetence. The compounds as defined above, are for use in prevention of surmenage/stress in a mammal.

In another aspect, the at least one additional therapeutic agent is selected from the group consisting of NSAID and opiates.

In one aspect, the additional therapeutic agent is an NSAID, such as butyl pyrrolidine, oxicams, propionic acid derivative, fenamic acid, coxibs and other non-steroidal anti-inflammatory and antirheumatic agents.

Examples of NSAIDs may include anti-inflammatory, antirheumatic and/or non-steroid NDSAIDs. NSAIDs may be selected from the group comprising or consisting of butylpyrazolidiner, such as fenylbutazon, or oxicams, such as meloxicam, or propionsyraderivat, such as ketoprofen, vedaprofen, carprofen and tepoxalin, or fenamater, such as tolfenamsyra, meklofenamsyra and flunixin, or coxiber, such as polmacoxib, firocoxib, robenacoxib, mavacoxib and cimicoxib, or other non-steroidal anti-inflammatory and antirheumatic agents, such as glukosaminoglykanpolysulfat, pentosanpolysulfat and grapiprant.

In one aspect, the additional therapeutic agent is an opiate, such as tramadol and tapentadol. In another aspect, the at least one additional therapeutic agent is an antirheumatic agent.

In one aspect, the invention relates to a pharmaceutical composition comprising (i) compound TT001, or hydrochloride or sulphate salt thereof, (ii) at least one agent selected from the group consisting NSAID, such as butyl pyrrolidine, oxicams, propionic acid derivative, fenamic acid, coxibs and other non-steroidal anti-inflammatory and antirheumatic agents and opiates, such as tramadol and tapentadol and (iii) a pharmaceutically acceptable excipient, carrier or diluent for use in prevention and/or treatment of surmenage/stress, in combination with one or more disorders selected from the group comprising or consisting of operative gastroesophageal reflux, anxiety, ulcer, renal and vascular disorders, wound healing and inappetence. The compounds as defined above, are for use in prevention of surmenage/stress in a mammal.

Such combination products employ the compounds as defined above within the dosage range described herein and the other pharmaceutically active compound or compounds within approved dosage ranges and/or the dosage described in the publication reference. In one aspect, the dosage range is 75wt%, or 50wt% of the prescribed dosage, when the compounds TT00, TT001 and/or TT002, as defined above, and the additional therapeutic agent are used in a combination composition.

The pharmaceutical composition comprising compounds TT00, TT001 and/orTT002 as defined above, optionally together with an additional agent as defined above, may be administered intravenous, intramuscular or orally. In one aspect the pharmaceutical composition is administered intravenous or intramuscular. TT001 can be administrated before, under and after the operation together with an anaesthetic protocol. In one aspect, said compounds may be administered intramuscularly.

In an aspect, compounds TT00, TT001 and/or TT002, or a pharmaceutically acceptable salt or solvate thereof, isotope or mixture thereof, for use pre- during- and/or past a situation in which the mammal experiences stress or is treated with one or more muscle relaxing drugs, such as an operation of a mammal, to reduce stress in the mammal. In an aspect, compounds TT00, TT001 and/or TT002, or a pharmaceutically acceptable salt or solvate thereof, isotope or mixture thereof are for use in shortening time for recuperation after operation in a mammal, wherein the time is shortened by at least 1 hour compared to no use of TT00, TT001 and/or TT002, pre- during- and/or past-operation.

The invention also relates to compounds TT00, TT001 and/or TT002, or a pharmaceutically acceptable salt or solvate thereof, isotope or mixture thereof, for use in prevention and/or treatment of stress in combination with operative gastroesophageal reflux combined with anxiety and/or pain in association with an operation, i.e. operative gastroesophageal reflux combined with anxiety in association with an operation, or operative gastroesophageal reflux combined with pain in association with an operation, or operative gastroesophageal reflux combined with anxiety and pain in association with an operation. The reflux may be pre-operative gastroesophageal reflux, intra-operative gastroesophageal reflux and/or post-operative gastroesophageal reflux.

The invention also relates to compounds TT00, TT001 and/or TT002, or a pharmaceutically acceptable salt or solvate thereof, isotope or mixture thereof, for use in prevention and/or treatment of stress in combination with ulcer, renal and/or vascular disorders in association with a situation in which the mammal experiences stress or is treated with one or more muscle relaxing drugs, such as an operation.

### Detailed description of various embodiments of the invention

The definitions set forth in this application are intended to clarify terms used throughout this application. The term "herein" means the entire application.

As used herein, "recuperation" means recovery of or restoration to the normal state of health and function.

It is to be understood that the expression " compounds TT00, TT001 and/or TT002*"* includes pharmaceutically acceptable salt, solvate, isotope or mixture thereof, unless specified otherwise.

As used herein, "pharmaceutically acceptable salts" refer to forms of the disclosed compounds, wherein the parent compound is modified by making acid or base salts thereof. Examples of pharmaceutically acceptable salts include, but are not limited to, mineral or organic acid salts of basic residues such as amines; alkali or organic salts of acidic residues, such as carboxylic acids; and the like. The pharmaceutically acceptable salts include the conventional non-toxic salts or the quaternary ammonium salts of the parent compound formed, for example, from non-toxic inorganic or organic acids. Such conventional non-toxic salts include those derived from inorganic acids such as hydrochloric acid. Examples of salts are hydrochloride salts or sulphate salts, expecially 4-[5-[(1*R*)-1-[5-(3-Chlorophenyl)-3-isoxazolyl]ethoxy]-4-methyl-4*H*-1,2,4-triazol-3-yl]pyridine hydrochloride or 4-[5-[(1*R*)-1-[5-(3-Chlorophenyl)-3-isoxazolyl]ethoxy]-4-methyl-4*H*-1,2,4-triazol-3-yl]pyridine sulphate.

The pharmaceutically acceptable salts of the present invention can be synthesized from the parent compound that contains a basic or acidic moiety by conventional chemical methods.

Generally, such salts can be prepared by reacting the free acid or base forms of these compounds with a stoichiometric amount of the appropriate base or acid in water or in an organic solvent, or in a mixture of the two; generally, non-aqueous media like diethyl ether, ethyl acetate, ethanol, isopropanol, or acetonitrile are used.

As used herein, the phrase "or pharmaceutically acceptable salts" include hydrates and solvates thereof.

As used herein, "operation" means a comatose and none-comatose operation, whereby the mammal is unconscious or conscious. The operation may be surgery. As used herein, "under operational condition(s)" means performing an operation on a mammal and includes a period before the operation until a period after the operation, whereby the period before the operation may be 1 to 4 days and the period after the operation may be 1 to 21 days. The term thus includes the whole period in which a mammal experiences stress due to the performance of an operation on the mammal.

As used herein "surmenage " or "stress" or "situation in which the mammal experiences stress" refers to a clinical condition of the mammal, wherein a clinician would diagnose one or more stress symptoms present in the mammal.

As used herein, "TLESR" means transient lower esophageal sphincter relaxation. During relaxation of the lower esophageal sphincter, fluid from the stomach can pass into the esophagus. This event is referred to as "reflux".

As used herein, "wound healing" means poor or reduced wound healing, i.e. a healing of a wound in a mammal that takes more time compared to the norm.

As used herein, "mammal" may include any mammal. For some disorders or combination of disorders, "mammal" may even include human. In sone aspects, "mammal" means dogs, pigs, cats or horses, rabbits, guinea pig, rat, birds, mice and cows. In other aspects, "mammal" means dogs, pigs, cats or horses.

Compounds TT00, TT001 and/or TT002, especially TT001, as defined herein may be isotopically labelled (or "radio-labelled"). In that instance, one or more atoms are replaced by an atom having an atomic mass or mass number different from the atomic mass or mass number typically found in nature (i.e., naturally occurring). Examples of suitable isotopes that may be incorporated include ²H (also written as "D" for deuterium), ³H (also written as "T" for tritium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I. The radionuclide that is used will depend on the specific application of that radio-labelled derivative. For example, for *in vitro* receptor labelling and competition assays, compounds that incorporate ³H or ¹⁴C are often useful. For radio-imaging applications ¹¹C or ¹⁸F are often useful. In some embodiments, the radionuclide is ³H. In some embodiments, the radionuclide is ¹⁴C. In some embodiments, the radionuclide is ¹¹C. And in some embodiments, the radionuclide is ¹⁸F. The present invention includes any isotope for use in diagnosis on mammals, such as dogs, pigs, cats or horses.

Compounds TT00, TT001 and/or TT002, especially TT001, may be administered orally, parenteral, buccal, vaginal, rectal, inhalation, insufflation, sublingually, intramuscularly, subcutaneously, topically, intranasally, intraperitoneally, intrathoracically, intravenously, epidurally, intrathecally, intracerebroventricularly and by injection into the joints.

The optimum dosage and frequency of administration will depend on the particular condition being treated and its severity; the species, age, sex, size and weight, diet, and general physical condition of the particular mammal; other medication the mammal may be taking; the route of administration; the formulation; and various other factors known to physicians and others skilled in the art.

The quantity of the compounds TT00, TT001 and/or TT002, especially TT001, to be administered will vary for the mammal being treated and will vary from about 0.01 ng/kg of body weight to 10 mg/kg of body weight per day, or 0.1 ng/kg to 1 mg/kg. For instance, dosages can be readily ascertained by those skilled in the art from this disclosure and the knowledge in the art. Thus, the skilled artisan can readily determine the amount of compound and optional additives, vehicles, and/or carrier in compositions to be administered in methods mentioned herein.

Compounds TT00, TT001 and/or TT002 can be prepared as a free base or a pharmaceutically acceptable salt or solvate thereof by the processes described in US7,476,684B2 or WO2007/040982A1, which are hereby included by reference.

For preparing pharmaceutical compositions from the compounds TT00, TT001 and/or TT002, especially TT001, inert, pharmaceutically acceptable carriers can be either solid or liquid. Solid form compositions include powders, tablets, dispersible granules, capsules, cachets, and suppositories. A solid carrier can be one or more substances, which may also act as diluents, flavoring agents, solubilizers, lubricants, suspending agents, binders, or tablet disintegrating agents; it can also be an encapsulating material.

Liquid form compositions include ointments, creams, gels, aqueous liquids, which may be formulated inside a transdermal patch.

A process for preparation of a capsule may comprise the following steps;
a) Mixing compound TT00, TT001 or TT002, especially TT001 together with additives, such as calcium hydrogen, phosphate and hydroxy propyl cellulose and stir for a period,
b) Adding further additives, such as mannitol and croscarmellose sodium,
c) Adding water and granulating the mixture,
d) Drying the obtained granulate,
e) Milling the dried granulate,
f) Adding further additives, such as sodium stearyl fumarate,
g) Mixing the obtained mixture,
h) Filling of the mixture in capsules or pressing the mixture into tablets.

### Pharmaceutical composition

### OINTMENT

TT001 Weight: 0.1-100 milligrams
propylene carbonate weight: 50 milligrams
paraffin, hard weight: 30 milligrams
beeswax white weight: 35 milligrams
paraffin, liquid weight: 110 milligrams
paraffin, white soft weight: 774.7 milligrams

### CREAM

TT001 Weight: 0.1-100 milligrams
propylene glycol weight: 100 milligrams
isopropyl myristate weight: 50 milligrams
cetostearyl alcohol weight: 52.5 milligrams
citric acid, monohydrate (e330) weight: 0.5 milligrams
disodium phosphate, anhydrous weight: 0.6 milligrams
water weight: a sufficient amount is added to achieve the target weight of 30 or 100 grams
paraffin, liquid weight: 400 milligrams
macrogol cetostearyl ether weight: 7.5 milligrams
disodium phosphate dodecahydrate (e339) weight: 1.5 milligrams
imidurea Weight: 2 milligrams

### FORMULATION METHOD FOR THE PREPARATION OF TT001 FOR I.V. DOSING, 0.1 to 10 mg/ml

The formulation method is applicable at concentrations in formulation between 0.1 and 10 mg/mL corresponding to 0.262 and 26.2 µmol/mL
TT001 M.W.: 381.8 g/mol

The dissolution of TT001 is moderate, allow a couple of hours for complete dissolution.

### VEHICLE

Preparation of 40 % w/v HPβCD solution in water for injection
Excipients Hydroxypropyl-β-cyclodextrin, Kleptose, Roquette (HPβCD) 400 mg (40 % w/v) Water for injection to 1 mL (1.13 g)
Appearance Clear
Density 1.13 g/cm3

### FORMULATION

Preparation of TT001 I.V. formulations between 0.1 and 10 mg/ml
TT001 parent form 0.1-10 mg
Vehicle (40 % w/v HPβCD solution in water for injection) to 1 mL (1.13 g)
Appearance Clear
Density 1.13 g/cm3

### Comments

The dissolution of TT001 is moderate, allow a couple of hours for complete dissolution.

### OINTMENT

Active corresponding to TT001 Weight: 0.1 to 50 milligrams
propylene carbonate weight: 50 milligrams
paraffin, hard weight: 30 milligrams
beeswax white weight: 35 milligrams
paraffin, liquid weight: 110 milligrams
paraffin, white soft weight: 774.7 milligrams

### CREAM

TT001 Weight: 0.1-100 milligrams
propylene glycol weight: 100 milligrams
isopropyl myristate weight: 50 milligrams
cetostearyl alcohol weight: 52.5 milligrams
citric acid, monohydrate (e330) weight: 0.5 milligrams
disodium phosphate, anhydrous weight: 0.6 milligrams
water weight: a sufficient amount is added to achieve the target weight of 30 or 100 grams
paraffin, liquid weight: 400 milligrams
macrogol cetostearyl ether weight: 7.5 milligrams
disodium phosphate dodecahydrate (e339) weight: 1.5 milligrams
imidurea weight: 2 milligrams

### CAPSULE

**Table 1. Components and quantities for TT001 Capsules 2 mg and 8 mg**

| Components | 2 mg | 8 mg | Function | Standard |
|---|---|---|---|---|
| TT001 | 2 mg | 8 mg | Drug Substance | AstraZeneca |
| Calcium hydrogen phosphate dehydrate/ Dibasic Calcium Phosphate Dihydrate | 70.5 mg | 68.5 mg | Filler | Ph Eur or USP |
| Hydroxypropylcellulose/ Hydroxypropyl Cellulose | 12 mg | 12 mg | Binder | Ph Eur or NF |
| Mannitol | 141.1 mg | 137.1 mg | Filler | Ph Eur or USP |
| Croscarmellose sodium | 9.6 mg | 9.6 mg | Disintegrant | Ph Eur or NF |
| Sodium stearyl fumarate | 4.8 mg | 4.8 mg | Lubricant | Ph Eur or NF |
| Water, purified/ Purified water^{a} | q.s. | q.s. | Granulation liquid | Ph Eur or USP |
| Capsules | 1 capsule | 1 capsule | Capsule | JP |

### Example 1

In cat or dog undergoing elective castration or ovariohysterectomy TT001 has a positive effect on duration of the recovery period.

To study recovery after neutering, cage demeanour scoring using a simple descriptive scale was used. The dogs were premedicated with acepromazine and pethidine, intermuscular (i.m.) in a first group, or in a second group TT001 was also added as pre-medication.

Twenty to forty minutes after pre-medication, the sedation was scored with the SDS (simple descriptive scale). The dogs were induced with propofol. Anesthesia was maintained with isoflurane in oxygen. Intraoperative analgesia was provided with morphine, prior to surgery. At the end of the surgery, group 1 received meloxicam, while group 2 received no additional treatment.

At the end of the procedure and recovery SDS was scored.

Group 2 showed the following;
Time to extubation became shorter,
Time to lifting the head after surgery became shorter,
Time to raising from the bed after surgery became shorter,
Time to start of spontaneous/voluntary urination after surgery became shorter,
Less nausea,
Time to starting to eat after surgery became shorter, and
Lower cortisol values than with commonly used anesthesia protocol.

### Example 2

In cat or dog undergoing elective castration or ovariohysterectomy TT001 has a positive effect on duration for the recovery period.

To study recovery after neutering, cage demeanour scoring using a simple descriptive scale was used. The dogs were premedicated with acepromazine and pethidine, i.m. in a first group, and meloxicam orally or in a second group TT001 was also added as pre-medication.

Twenty to forty minutes after pre-medication the sedation was scored with the SDS (simple descriptive scale). The dogs were induced with propofol. Anesthesia was maintained with isoflurane in oxygen. Intraoperative analgesia was provided with morphine, prior to surgery. At the end of the surgery, group 1 continued to receive meloxicam, while group 2 continued to receive TT001 as well as meloxicam. No additional treatment was administered.

At the end of the procedure and recovery SDS was scored.

Group 2 showed the following;
Time to extubation became shorter than for group 1,
Time to lifting the head after surgery became shorter than for group 1,
Time to raising from the bedding after surgery became shorter than for group 1,
Time to start of spontaneous/voluntary urination after surgery became shorter than for group 1,
Less nausea than for group 1,
Time to starting to eat after surgery became shorter than for group 1,
Lower cortisol values than in group 1, and
Less time in intensive care/PACU unit than for group 1.

In this study, TT001 displayed no effect on LES pressure. There was also no significant effect on swallows observed with TT001 in this study, however, there was a numerical trend towards a decrease in swallows for the higher dosages, which can be reconciled with a CNS mediated effect.

### Example 3, Forced Swim Test

TT001 has been used in the Forced Swim Test. _For protocol see Can A., et al., The Mouse Forced Swim Test, J Vis Exp. 2012; (59): 3638.

The results in table 1 show that the test animals are still for a significantly shorter period than placebo group.

**Table 1 Effects of TT001 on mouse FST behavior**

| Treatment (µmol/kg) | Immobility duration (sec) |
|---|---|
| Vehicle | 112.5 ± 14.10 |
| TT001 (3) | 51.15 ± 7.788 ^{a} |
| TT001 (10) | 47.45 ± 11.45 ^{a} |
| TT001 (30) | 44.83 ± 4.693 ^{a} |

| | |
|---|---|
| ^{a} p<0.01 is associated with one way ANOVA comparing TT001 vs vehicle. | |

This test shows that TT001 reduces stress. Besides, none of the test animals had signs of gastric ulcer or even any changes in mucosa.

### Example 4, Learned helplessness studies

Nomura S. et al., Studies on animal model of depression: review and perspective, Review, Yakubutsu Seishin Kodo, 1989 Dec;9(4):349-58.

TT001 was used in learned helplessness studies in rats. The results are shown in table 2.

**Table 2 Escape Failures Following Each Treatment level**

| Treatment | | Day 3 | | Day 4 | | Total |
|---|---|---|---|---|---|---|
| Compound | Dose | Mean | SEM | Mean | SEM | Day 3 + Day 4 |
| Vehicle | 0 | 23.2 | 7.8 | 11.1 | 6.20 | 34.25 |
| TT001 | 1.8 µmol/kg | 6.9 | 2.5 | 2.2 | 1.27 | 9.1 ^{a} |

| | | | | | | |
|---|---|---|---|---|---|---|
| p < 0.05 by t-test vs. vehicle group. | | | | | | |

The results show that TT001 lowered the stress and that stomach ulcers did not appear. Thus, TT001 has a specific therapeutic effect on stress and gastric ulcer.

### Example 5, Stress and lab-work - Ulcer

Toxicological studies for TT001 in dogs, were performed on dogs using 2, 6, 7 or 31 mg/kg, for a maximum of 52 weeks. The dogs were fed with gavage throughout this period and blood samples were taken throughout the period.

Balcome at al Contemporary Topics 2004 by the American Association for Laboratory Animal Science. Vol 43. No.6/November 2004, showed that gavage and regular blood sampling create a lot of stress in the animals and an increase in cortisol values is normal under these circumstances. In addition, the animals are separated and live in small areas which further increase the stress.

After 52 weeks, none of the animals showed signs of gastric ulcer despite the fact that the environment and handling during the 52 weeks was causing stress. The results show that TT001 is a substance that relieves stress and thus the risk of stomach ulcers.

## Claims

1. Compounds 4-[5-[(rac)-1-[5-(3-Chlorophenyl)-3-isoxazolyl]ethoxy]-4-methyl-4*H*-1,2,4-triazol-3-yl]pyridine (TT00), or 4-[5-[(1R)-1-[5-(3-Chlorophenyl)-3-isoxazolyl]ethoxy]-4-methyl-4*H*-1,2,4-triazol-3-yl]pyridine (TT001) and/or 4-[5-[(15)-1-[5-(3-Chlorophenyl)-3-isoxazolyl]ethoxy]-4-methyl-4*H*-1,2,4-triazol-3-yl]pyridine (TT002), or a pharmaceutically acceptable salt, solvate, isotope, or mixture thereof, for use in prevention and/or treatment of stress in combination with one or more disorders selected from the group consisting of operative gastroesophageal reflux, anxiety, ulcer, renal and vascular disorders, wound healing and inappetence in a dog, cat, horse or pig.

2. Compounds TT00, TT001 and/or TT002, or a pharmaceutically acceptable salt, solvate, isotope, or mixture thereof, for use according to claim 1, wherein the dog, cat, horse or pig is under surgical operation conditions.

3. Compounds TT00, TT001 and/or TT002, or a pharmaceutically acceptable salt, solvate, isotope, or mixture thereof, for use according to claims 1 or 2, wherein the dog, cat, horse or pig is treated with one or more muscle relaxing drugs.

4. Compounds TT00, TT001 and/or TT002, or a pharmaceutically acceptable salt, solvate, isotope, or mixture thereof, for use according to claims 2 or 3, wherein said compounds are administered prior to the operation.

5. Compounds TT00, TT001 and/or TT002, or a pharmaceutically acceptable salt, solvate, isotope, or mixture thereof, for use according to claim 2 or 3, wherein said compounds are administered after the operation.

6. Compounds TT00, TT001 and/or TT002, or a pharmaceutically acceptable salt, solvate, isotope, or mixture thereof, for use according to any one of claims 1 to 5 in combination with prevention and/or treatment of ulcer, renal and/or vascular disorders.

7. Compounds TT00, TT001 and/or TT002, or a pharmaceutically acceptable salt, solvate, isotope, or mixture thereof, for use according to any one of claims 1 to 5 in combination with prevention and/or treatment of wound healing and/or inappetence.

8. Compounds TT00, TT001 and/or TT002, or a pharmaceutically acceptable salt, solvate, isotope, or mixture thereof, for use according to any one of claims 1 to 5 in combination with prevention and/or treatment of operative gastroesophageal reflux.

9. Compounds TT00, TT001 and/or TT002, or a pharmaceutically acceptable salt, solvate, isotope, or mixture thereof, for use according to any one of claims 1 to 5 in combination with anxiety.

10. A pharmaceutical composition, comprising compounds TT00, TT001 and/or TT002, or a pharmaceutically acceptable salt, solvate, isotope, or mixture thereof, in the association with a pharmaceutically acceptable adjuvant, diluent or carrier, for use in prevention and/or treatment of stress in combination with one or more disorders selected from the group comprising operative gastroesophageal reflux, anxiety, ulcer, renal and vascular disorders, wound healing and inappetence in a dog, cat, horse or pig.

11. The pharmaceutical composition for use according to claim 10, comprising (i) compounds TT00, TT001 and/or TT002, or a pharmaceutically acceptable salt, solvate or isotope or mixture thereof, (ii) an additional therapeutic agent, or a pharmaceutically acceptable salt or solvate thereof, and (iii) a pharmaceutically acceptable excipient, carrier or diluent.

12. The pharmaceutical composition for use according to claim 10, comprising (i) compounds TT00, TT001 and/or TT002, or a pharmaceutically acceptable salt, solvate or isotope or mixture thereof, and a pharmaceutically acceptable excipient, carrier or diluent (ii) an additional therapeutic agent, or a pharmaceutically acceptable salt or solvate thereof, and a pharmaceutically acceptable excipient, carrier or diluent.

13. The pharmaceutical composition for use according to claim 10 to 12, wherein the additional therapeutic agent is an NSAID selected from the group comprising butyl pyrrolidine, oxicams, propionic acid derivative, fenamic acid, coxibs and other non-steroidal anti-inflammatory, an opiate selected from the group comprising tramadol and tapentadol, or an antirheumatic agent.

14. Compounds TT00, TT001 and/or TT002, or a pharmaceutically acceptable salt, solvate, isotope, or mixture thereof, for use according to any one of claims 1 to 9, or the pharmaceutical composition according to claim 10 to 13, wherein the compound is TT001, or a hydrochloride salt thereof.

15. Compounds TT00, TT001 and/or TT002, or a pharmaceutically acceptable salt, solvate, isotope, or mixture thereof, for use according to any one of claims 1 to 9, 14 or the pharmaceutical composition according to claim 10 to 14, for use in a dog.

## Patentansprüche

1. Verbindungen 4-[5-[(rac)-1-[5-(3-Chlorphenyl)-3-isoxazolyl]ethoxy]-4-methyl-4*H*-1,2,4-triazol-3-yl]pyridin (TT00) oder 4-[5-[(1*R*)-1-[5-(3-Chlorphenyl)-3-isoxazolylethoxy]-4-methyl-4*H*-1,2,4-triazol-3-yl]pyridin (TT001) und/oder 4-[5-[(1*S*)-1-[5-(3-Chlorphenyl)-3-isoxazolyl]ethoxy]-4-methyl-4*H*-1,2,4-triazol-3-yl]pyridin (TT002) oder ein pharmazeutisch unbedenkliches Salz, Solvat, Isotop oder Mischung davon, zur Verwendung bei einer Vorbeugung und/oder einer Behandlung von Stress in Kombination mit einem oder mehreren Erkrankungen, die aus der Gruppe ausgewählt sind, bestehend aus operativem gastroösophagealem Reflux, Unruhe, Geschwür-, Nieren- und Gefäßerkrankungen, Wundheilung und Appetitmangel in einem Hund, einer Katze, einem Pferd oder einem Schwein.

2. Verbindungen TT00, TT001 und/oder TT002 oder ein pharmazeutisch unbedenkliches Salz, Solvat, Isotop oder Mischung davon zur Verwendung nach Anspruch 1, wobei sich der Hund, die Katze, das Pferd oder das Schwein unter chirurgischen Operationsbedingungen befindet.

3. Verbindungen TT00, TT001 und/oder TT002 oder ein pharmazeutisch unbedenkliches Salz, Solvat, Isotop oder Mischung davon zur Verwendung nach den Ansprüchen 1 oder 2, wobei der Hund, die Katze, das Pferd oder das Schwein mit einem oder mehreren Muskelentspannungsmedikamenten behandelt wird.

4. Verbindungen TT00, TT001 und/oder TT002 oder ein pharmazeutisch unbedenkliches Salz, Solvat, Isotop oder Mischung davon zur Verwendung nach den Ansprüchen 2 oder 3, wobei die Verbindungen vor der Operation verabreicht werden.

5. Verbindungen TT00, TT001 und/oder TT002 oder ein pharmazeutisch unbedenkliches Salz, Solvat, Isotop oder Mischung davon zur Verwendung nach Anspruch 2 oder 3, wobei die Verbindungen nach der Operation verabreicht werden.

6. Verbindungen TT00, TT001 und/oder TT002 oder ein pharmazeutisch unbedenkliches Salz, Solvat, Isotop oder Mischung davon zur Verwendung nach einem der Ansprüche 1 bis 5 in Kombination mit der Vorbeugung und/oder der Behandlung von Geschwür-, Nieren- und/oder Gefäßerkrankungen.

7. Verbindungen TT00, TT001 und/oder TT002 oder ein pharmazeutisch unbedenkliches Salz, Solvat, Isotop oder Mischung davon zur Verwendung nach einem der Ansprüche 1 bis 5 in Kombination mit der Vorbeugung und/oder der Behandlung der Wundheilung und/oder des Appetitmangels.

8. Verbindungen TT00, TT001 und/oder TT002 oder ein pharmazeutisch unbedenkliches Salz, Solvat, Isotop oder Mischung davon zur Verwendung nach einem der Ansprüche 1 bis 5 in Kombination mit der der Vorbeugung und/oder der Behandlung des operativen gastroösophagealen Refluxes.

9. Verbindungen TT00, TT001 und/oder TT002 oder ein pharmazeutisch unbedenkliches Salz, Solvat, Isotop oder Mischung davon zur Verwendung nach einem der Ansprüche 1 bis 5 in Kombination mit Unruhe.

10. Pharmazeutische Zusammensetzung, umfassend Verbindungen TT00, TT001 und/oder TT002 oder ein pharmazeutisch unbedenkliches Salz, Solvat, Isotop oder Mischung davon, in der Assoziation mit einem pharmazeutisch unbedenklichen Adjuvans, Verdünnungsmittel oder Träger, zur Verwendung bei der Vorbeugung und/oder der Behandlung von Stress in Kombination mit einem oder mehreren Erkrankungen, die aus der Gruppe ausgewählt sind, umfassend operativen gastroösophagealen Reflux, Unruhe, Geschwür-, Nieren- und Gefäßerkrankungen, Wundheilung und Appetitmangel in einem Hund, einer Katze, einem Pferd oder einem Schwein.

11. Pharmazeutische Zusammensetzung zur Verwendung nach Anspruch 10, umfassend (i) Verbindungen TT00, TT001 und/oder TT002 oder ein pharmazeutisch unbedenkliches Salz, Solvat oder Isotop oder Mischung davon, (ii) ein zusätzliches therapeutisches Mittel oder ein pharmazeutisch unbedenkliches Salz oder Solvat davon und (iii) einen pharmazeutisch unbedenklichen Arzneistoffträger, einen pharmazeutisch unbedenklichen Träger oder ein pharmazeutisch unbedenkliches Verdünnungsmittel.

12. Pharmazeutische Zusammensetzung zur Verwendung nach Anspruch 10, umfassend (i) Verbindungen TT00, TT001 und/oder TT002 oder ein pharmazeutisch unbedenkliches Salz, Solvat oder Isotop oder Mischung davon und einen pharmazeutisch unbedenklichen Arzneistoffträger, einen pharmazeutisch unbedenklichen Träger oder ein pharmazeutisch unbedenkliches Verdünnungsmittel, (ii) ein zusätzliches therapeutisches Mittel oder ein pharmazeutisch unbedenkliches Salz oder Solvat davon und einen pharmazeutisch unbedenklichen Arzneistoffträger, einen pharmazeutisch unbedenklichen Träger oder ein pharmazeutisch unbedenkliches Verdünnungsmittel.

13. Pharmazeutische Zusammensetzung zur Verwendung nach Anspruch 10 bis 12, wobei das zusätzliche therapeutische Mittel ein NSAID ist, das aus der Gruppe ausgewählt ist, umfassend Butylpyrrolidin, Oxicame, Propionsäurederivat, Fenaminsäure, Coxibe und andere nicht steroidale entzündungshemmende, ein Opiat, das aus der Gruppe ausgewählt ist, umfassend Tramadol und Tapentadol oder einem Antirheumatikum.

14. Verbindungen TT00, TT001 und/oder TT002 oder ein pharmazeutisch unbedenkliches Salz, Solvat, Isotop oder Mischung davon zur Verwendung nach einem der Ansprüche 1 bis 9 oder die pharmazeutische Zusammensetzung nach Anspruch 10 bis 13, wobei die Verbindung TT001 oder ein Hydrochloridsalz davon ist.

15. Verbindungen TT00, TT001 und/oder TT002 oder ein pharmazeutisch unbedenkliches Salz, Solvat, Isotop oder Mischung davon zur Verwendung nach einem der Ansprüche 1 bis 9, 14 oder die pharmazeutische Zusammensetzung nach Anspruch 10 bis 14 zur Verwendung in einem Hund.

## Revendications

1. Composés 4-[5-[(rac)-1-[5-(3-chlorophényl)-3-isoxazolyl]éthoxy]-4-méthyl-4H-1,2,4-triazol-3-yl]pyridine (TT00), ou 4-[5-[(1 R)-1-[5-(3-chlorophényl)-3-isoxazolyl]éthoxy]-4-méthyl-4H-1,2,4-triazol-3-yl]pyridine (TT001) et/ou 4-[5-[(1*S*)-1-[5-(3-chlorophényl)-3-isoxazolyl]éthoxy]-4-méthyl-4*H*-1,2,4-triazol-3-yl]pyridine (TT002), ou sel pharmaceutiquement acceptable, solvate, isotope, ou mélange de ceux-ci, pour une utilisation dans la prévention et/ou le traitement de stress en combinaison avec un ou plusieurs troubles choisis dans le groupe constitué de reflux gastro-oesophagien opératoire, anxiété, ulcère, troubles rénaux et vasculaires, cicatrisation de plaie et inappétence chez un chien, un chat, un cheval ou un cochon.

2. Composés TT00, TT001 et/ou TT002, ou sel pharmaceutiquement acceptable, solvate, isotope, ou mélange de ceux-ci, pour une utilisation selon la revendication 1, le chien, le chat, le cheval ou le cochon étant dans des conditions d'opération chirurgicale.

3. Composés TT00, TT001 et/ou TT002, ou sel pharmaceutiquement acceptable, solvate, isotope, ou mélange de ceux-ci, pour une utilisation selon les revendications 1 ou 2, le chien, le chat, le cheval ou le cochon étant traité avec un ou plusieurs médicaments myorelaxants.

4. Composés TT00, TT001 et/ou TT002, ou sel pharmaceutiquement acceptable, solvate, isotope, ou mélange de ceux-ci, pour une utilisation selon les revendications 2 ou 3, lesdits composés étant administrés avant l'opération.

5. Composés TT00, TT001 et/ou TT002, ou sel pharmaceutiquement acceptable, solvate, isotope, ou mélange de ceux-ci, pour une utilisation selon la revendication 2 ou 3, lesdits composés étant administrés après l'opération.

6. Composés TT00, TT001 et/ou TT002, ou sel pharmaceutiquement acceptable, solvate, isotope, ou mélange de ceux-ci, pour une utilisation selon l'une quelconque des revendications 1 à 5 en combinaison avec la prévention et/ou le traitement d'ulcère, de troubles rénaux et/ou vasculaires.

7. Composés TT00, TT001 et/ou TT002, ou sel pharmaceutiquement acceptable, solvate, isotope, ou mélange de ceux-ci, pour une utilisation selon l'une quelconque des revendications 1 à 5 en combinaison avec la prévention et/ou le traitement de cicatrisation de plaie et/ou d'inappétence.

8. Composés TT00, TT001 et/ou TT002, ou sel pharmaceutiquement acceptable, solvate, isotope, ou mélange de ceux-ci, pour une utilisation selon l'une quelconque des revendications 1 à 5 en combinaison avec la prévention et/ou le traitement de reflux gastro-oesophagien opératoire.

9. Composés TT00, TT001 et/ou TT002, ou sel pharmaceutiquement acceptable, solvate, isotope, ou mélange de ceux-ci, pour une utilisation selon l'une quelconque des revendications 1 à 5 en combinaison avec l'anxiété.

10. Composition pharmaceutique, comprenant des
composés TT00, TT001 et/ou TT002, ou un sel pharmaceutiquement acceptable, solvate, isotope, ou mélange de ceux-ci, en association avec un adjuvant, diluant ou véhicule pharmaceutiquement acceptable, pour une utilisation dans la prévention et/ou le traitement de stress en combinaison avec un ou plusieurs troubles choisis dans le groupe comprenant reflux gastro-oesophagien opératoire, anxiété, ulcère, troubles rénaux et vasculaires, cicatrisation de plaie et inappétence chez un chien, un chat, un cheval ou un cochon.

11. Composition pharmaceutique pour une utilisation selon la revendication 10, comprenant (i) des composés TT00, TT001 et/ou TT002, ou un sel pharmaceutiquement acceptable, solvate ou isotope ou mélange de ceux-ci, (ii) un agent thérapeutique supplémentaire, ou un sel pharmaceutiquement acceptable ou solvate de celui-ci, et (iii) un excipient, véhicule ou diluant pharmaceutiquement acceptable.

12. Composition pharmaceutique pour une utilisation selon la revendication 10, comprenant (i) des composés TT00, TT001 et/ou TT002, ou un sel pharmaceutiquement acceptable, solvate ou isotope ou mélange de ceux-ci, et un excipient, véhicule ou diluant pharmaceutiquement acceptable (ii) un agent thérapeutique supplémentaire, ou un sel pharmaceutiquement acceptable ou solvate de celui-ci, et un excipient, véhicule ou diluant pharmaceutiquement acceptable.

13. Composition pharmaceutique pour une utilisation selon la revendication 10 à 12, dans laquelle l'agent thérapeutique supplémentaire est un AINS choisi dans le groupe comprenant butylpyrrolidine, oxicams, dérivé d'acide propionique, acide fénamique, coxibs et autre anti-inflammatoire non stéroïdien, un opiacé choisi dans le groupe comprenant tramadol et tapentadol, ou un agent antirhumatismal.

14. Composés TT00, TT001 et/ou TT002, ou sel pharmaceutiquement acceptable, solvate, isotope, ou mélange de ceux-ci, pour une utilisation selon l'une quelconque des revendications 1 à 9, ou composition pharmaceutique selon la revendication 10 à 13, le composé étant TT001 , ou un sel chlorhydrate de celui-ci.

15. Composés TT00, TT001 et/ou TT002, ou sel pharmaceutiquement acceptable, solvate, isotope, ou mélange de ceux-ci, pour une utilisation selon l'une quelconque des revendications 1 à 9, 14 ou composition pharmaceutique selon la revendication 10 à 14, pour une utilisation chez un chien.
